# EUROPEAN PATENT SPECIFICATION

(11) **EP 3 408 404 B1**
(45) Date of publication and mention of the grant of the patent: **23.06.2021**
(21) Application number: 16763358.5
(22) Date of filing: 09.08.2016
(51) Int. Cl.: C12Q 1/22, A61L 2/07, C12M 1/12, A61L 2/28, C12M 1/00

(54) **BIOLOGICAL INDICATORS FOR STEAM STERILIZATION**
BIOLOGISCHE INDIKATOREN FÜR DAMPFSTERILISIERUNG
INDICATEURS BIOLOGIQUES POUR LA STÉRILISATION PAR VAPEUR

(30) Priority: 25.01.2016 US 201615005182
(43) Date of publication of application: 05.12.2018
(73) Proprietor: American Sterilizer Company, Mentor, OH 44060 (US)
(72) Inventor: YIRAVA, William A., Willoughby, Ohio 44094 (US); BEAHN, Nicole M., Mentor, Ohio 44060 (US); FRANCISKOVICH, Phillip P., Concord, Ohio 44077 (US); CREGGER, Tricia, Fairlawn, Ohio 44333 (US)
(74) Representative: Brand Murray Fuller LLP
(86) International application number: PCT/US2016/046144
(87) International publication number: WO 2017/131819

(56) References cited:
- WO-A1-99/53019
- US-A- 4 883 641
- US-A1- 2008 070 227
- Thereza Christina Vessoni Penna ET AL: "Effect of media on spore yield and thermal resistance of Bacillus stearothermophilus", Applied Biochemistry and Biotechnology, 1 March 2003 (2003-03-01), pages 287-294, XP055309625, Totowa DOI: 10.1385/ABAB:106:1-3:287 Retrieved from the Internet: URL:http://rd.springer.com/content/pdf/10. 1385/ABAB:106:1-3:287.pdf [retrieved on 2016-10-11]
- T J Berger ET AL: "The effect of formulation of parenteral solutions on microbial growth --measurement of D- and z-values", PDA journal of pharmaceutical science and technology / PDA, 1 January 1995 (1995-01-01), page 32, XP055309541, UNITED STATES Retrieved from the Internet: URL:http://journal.pda.org/content/49/1/32 .long [retrieved on 2016-10-11]
- WANG Y J ET AL: "OPTIMIZATION OF AUTOCLAVE CYCLES AND SELECTION OF FORMULATION FOR PARENTERAL PRODUCTS, PART III: EFFECT OF FORMULATION VARIABLES ON SPORICIDAL KINETICS", JOURNAL OF PARENTERAL SCIENCE AND TECHNOLOGY, PARENTAL DRUF ASSOCIATION, US, vol. 38, no. 2, 1 January 1984 (1984-01-01), pages 78-82, XP009035739, ISSN: 0279-7976
- Thereza Penna ET AL: "The effect of composition of parenteral solution on the thermal resistance of Bacillus stearothermophilus and Bacillus subtilis spores", Applied Biochemistry and Biotechnology, 1 March 2002 (2002-03-01), pages 539-551, XP055608744, Totowa DOI: 10.1385/ABAB:98-100:1-9:539 Retrieved from the Internet: URL:https://link.springer.com/content/pdf/ 10.1385/ABAB:98-100:1-9:539.pdf

## Description

### Technical Field

This invention relates to biological indicators and, more particularly, to biological indicators that are treated to reduce their resistance to steam.

### Background

Biological indicators, which typically comprise a carrier and test microorganisms deposited on the carrier, are used to monitor steam sterilization processes. The biological indicator is placed in a sterilization chamber and subjected to a steam sterilization cycle along with the load intended for sterilization (e.g., a medical device). Following the sterilization cycle, the biological indicator is exposed to a growth media and incubated for the purpose of determining if any of the test microorganisms are viable. A successful sterilization cycle results in a complete inactivation (no outgrowth) of the test microorganisms. An unsuccessful sterilization cycle results in an incomplete inactivation (outgrowth detected) of the test microorganisms.
WO 99/53019 discloses microbial spores comprising one or more additives specifically bound to sterilant-sensitive sites in the spores, wherein the one or more additives increase sensitivity to the spores to a sterilant.

### Summary

Biological indicators typically comprise standardized preparations of specific test microorganisms with known characteristics (e.g., a defined population, purity, resistance characteristic, etc.). The test microorganisms may be microorganisms capable of forming endospores. As such, the test microorganisms may be in the "spore state." A biological indicator may be prepared by depositing the test microorganisms from a spore crop onto a carrier. The carrier may comprise a substrate such as filter paper or an interior surface of a self-contained biological indicator (SCBI).

The prior art methods for producing biological indicators are typically contingent upon multiple components of the biological indicator producing consistent results to maintain predictable resistance levels. A problem in the art relates to the fact that at times unexplained results occur when using biological indicators that can be traced to inconsistencies with respect to the resistance of the biological indicator. With this invention it is possible to control biological indicator resistance levels to meet customer requirements without requiring excessive amounts of screening and selection of manufactured spores to find those that show the desired resistance levels. As such, with this invention, it is possible to use fewer batches of test microorganisms requiring cultivation to meet customer needs for providing biological indicators with desired resistance levels.

This invention relates to a biological indicator, comprising: a carrier inoculated with a test microorganism and an effective amount of a carbohydrate to reduce the resistance of the biological indicator to steam sterilization as defined in appended claims 1-6. This invention is advantageous for biological indicators wherein the test microorganisms exhibit resistance levels that are higher than desired for their anticipated end use.

In an embodiment, this invention relates to a steam sterilization process, comprising: exposing an article to be sterilized and the above-indicated biological indicator to steam.

In an embodiment, this invention relates to a process for determining the effectiveness of a steam sterilization process, comprising: exposing an article to be sterilized and the above-indicated biological indicator to steam; and incubating the biological indicator in the presence of a growth media to determine whether the sterilization process is effective.

### Brief Description of the Drawings

Fig. 1 is a graph showing data from Example 1 wherein steam resistance at 121°C is determined for two biological indicators derived from suspensions of *Geobacillus stearothermophilus* spores, one of the biological indicators being derived from a suspension of the spores in an aqueous xylose solution, and the other biological indicator being derived from a suspension of the spores in water without xylose.
Fig. 2 is a graph showing data from Example 2 wherein steam resistance at 132°C is determined for two biological indicators derived from suspensions of *Geobacillus stearothermophilus* spores, one of the biological indicators being derived from a suspension of the spores in an aqueous xylose solution, and the other biological indicator being derived from a suspension of the spores in water without xylose.
Figs. 3A and 3B are graphs showing data from Example 3 wherein steam resistance at 121°C is determined for four biological indicators derived from suspensions of *Geobacillus stearothermophilus* spores, one of the biological indicators being derived from a suspension of the spores in water (Fig. 3A), and three of the biological indicators being derived from suspensions of the spores in various aqueous solutions of xylose (Fig. 3B).
Fig. 4 is a graph showing data from Example 4 wherein steam resistance at 121°C is shown for three biological indicators derived from suspensions of *Geobacillus stearothermophilus* spores in water, a 0.5M solution of xylose, and a 0.5M solution of dextrose.
Fig. 5A is a schematic illustration of the biological indicator evaluation resistometer (BIER) vessel used in Examples 1-4.
Fig. 5B is a cycle graph showing phases 1-6 used with the BIER vessel in Examples 1-4.
Fig. 6 is a schematic illustration of a vial containing a dried and a rehydrated suspension of spores used as a biological indicator.
Fig. 7 is a perspective view of an SCBI which can be used in accordance with the present invention.
Fig. 8 is a cross-sectional view of the SCBI of Fig. 7 (taken along the line 8-8 in Fig. 7) showing a cap mounted on a container in a first non-activated position;
Fig. 9 is a cross-sectional view of the SCBI of Fig. 7 (taken along the line 8-8 in Fig. 7) showing the cap mounted on the container in a second activated position.
Fig. 10 is a cross-sectional view of the SCBI of Fig. 7 (taken along line 10-10 in Fig. 7) showing the indicator in a second activated position.
Fig. 11 is a side-top perspective view of a test pack that may be used with an SCBI in accordance with the invention.
Fig. 12 is a cross-sectional view of the test pack illustrated in Fig. 11 taken along lines 12-12 of Fig. 11.
Fig. 13 is a top plan view of a test pack that can be used in accordance with the present invention, with an SCBI in a first recessed compartment and a chemical integrator and/or chemical indicator in a second recessed compartment.

### Detailed Description

All ranges and ratio limits disclosed in the specification and claims may be combined in any manner. It is to be understood that unless specifically stated otherwise, references to "a," "an," and/or "the" may include one or more than one, and that reference to an item in the singular may also include the item in the plural.

The phrase "and/or" should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "X and/or Y," when used in conjunction with open-ended language such as "comprising" can refer, in one embodiment, to X without Y (optionally including elements other than Y); in another embodiment, to Y without X (optionally including elements other than X); in yet another embodiment, to both X and Y (optionally including other elements); etc.

The word "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," may refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

The phrase "at least one," in reference to a list of one or more elements, should be understood to mean at least one element selected from any one or more of the elements in the list of elements, but not necessarily including at least one of each and every element specifically listed within the list of elements and not excluding any combination of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of X and Y" (or, equivalently, "at least one of X or Y," or, equivalently "at least one of X and/or Y") can refer, in one embodiment, to at least one, optionally including more than one, X, with no Y present (and optionally including elements other than Y); in another embodiment, to at least one, optionally including more than one, Y, with no X present (and optionally including elements other than X); in yet another embodiment, to at least one, optionally including more than one, X, and at least one, optionally including more than one, Y (and optionally including other elements); etc.

The transitional words or phrases, such as "comprising," "including," "carrying," "having," "containing," "involving," "holding," and the like, are to be understood to be open-ended, i.e., to mean including but not limited to.

The term "inactivation" of a test organism (e.g., bacterial spores) refers to the loss of ability of the test organism to germinate, outgrow and/or multiply.

The term "log reduction" is a mathematical term to show the number of live test organisms (e.g., bacterial spores) inactivated by contacting the test organisms with a sterilant. A "4 log reduction" means that the number of live test organisms is 10,000 times smaller. A "5 log reduction" means that the number of live test organisms is 100,000 times smaller. A "6 log reduction" means that the number of live test organisms is 1,000,000 times smaller.

The term "sterilization" is often taken to refer to a process wherein a total absence of living test organisms is achieved. However, this term is also used herein to refer to processes that are less rigorous than sterilization processes. These may include, for example, disinfection, sanitization, decontamination, cleaning, and the like. The sterilization processes provided for herein may be conducted for an effective period of time to achieve at least about a 4 log reduction, or at least about a 5 log reduction, or at least about a 6 log reduction in the number of test organisms capable of germination, outgrowth and/or multiplication.

The term "biological indicator" refers to a microbiological test system which comprises a carrier and test organisms deposited on the carrier. The biological indicator may be used in combination with a process indicator.

The term "carrier" refers to a supporting material onto which test organisms may be deposited.

The term "inoculated carrier" refers to a carrier onto which test organisms have been deposited.

The term "test organism" refers to a microorganism which is more resistant to a sterilization process than the organisms to be destroyed by the sterilization process. The test organisms may comprise spores, for example, bacterial spores.

The term "D-value" or "decimal reduction value" refers to the time required to achieve inactivation of 90% of a population of test organisms (also known as a 1 log reduction). The D-value may be expressed in minutes.

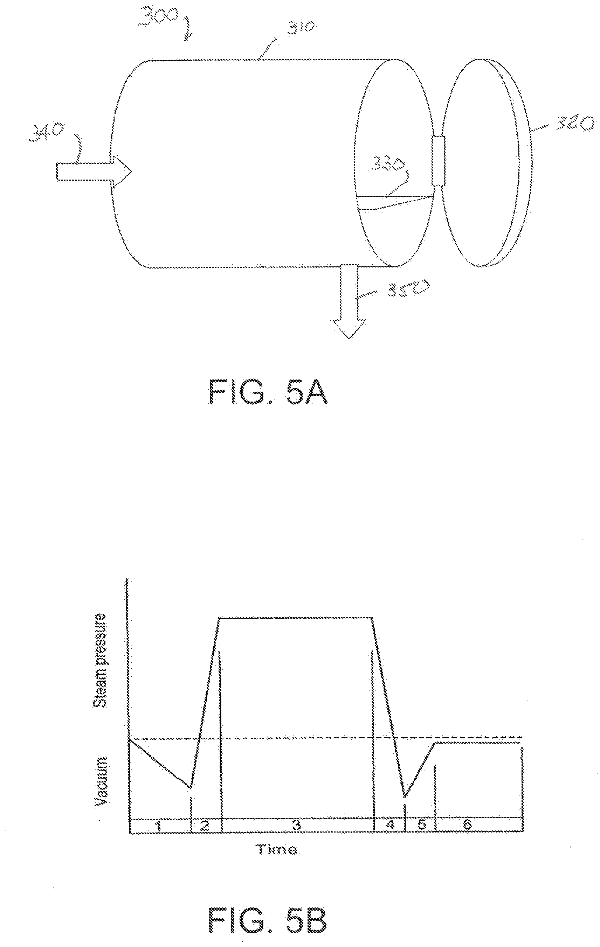

The term "Biological Indicator Evaluation Resistometer vessel" or "BIER vessel" refers to an apparatus that provides environmental conditions for evaluating the resistance of a biological indicator to steam sterilization. Temperature, pressure, and time are the primary variables which influence the rate of microbial destruction in the BIER vessel. The more resistant spores survive longer in the BIER vessel than the less resistant spores. A BIER vessel that may be used is illustrated in Fig. 5A.

The test organism may comprise bacterial spores. The test organism may comprise spores of the *Bacillus* or *Clostridia* genera. The test organism may comprise spores of *Geobacillus stearothermophilus, Bacillus atrophaeus, Bacillus sphaericus, Bacillus anthracis, Bacillus pumilus, Bacillus coagulans, Clostridium sporogenes, Clostridium difficile, Clostridium botulinum, Bacillus subtilis globigii, Bacillus cereus, Bacillus circulans,* or a mixture of two or more thereof. In the invention, the test organism comprises spores of *Geobacillus stearothermophilus.*

The carbohydrate may comprise a compound represented by the empirical formula Cₘ(H₂O)ₙ where m and n are numbers. The carbohydrate may comprise carbon, hydrogen and oxygen atoms, with a hydrogen:oxygen ratio of 2:1. The carbohydrate may be a saccharide. The saccharide may be a monosaccharide, disaccharide, oligosaccharide, polysaccharide, or a mixture of two or more thereof. The monosaccharides and disaccharides may be referred to as sugars. The monosaccharides may be particularly useful. The monosaccharides may include xylose, glucose (dextrose), fructose, galactose, arabinose, ribulose, mannose, or a mixture of two or more thereof. The monosaccharide may comprise triose, tetrose, pentose, hexose, heptose, octose, nonose, or a mixture of two or more thereof. Pentose may be particularly useful. The carbohydrates may be obtained from sugar cane, sugar beet, corn syrup, and the like. The carbohydrates may be synthetically made. Nonose may be synthetic as are some diet based sugar alcohols which may be used. In the invention, the carbohydrate is xylose or dextrose.

The biological indicator may be used with any steam sterilization process. The biological indicator along with the articles to be sterilized may be exposed to steam during the sterilization process. The steam sterilization process may be conducted in a steam sterilization chamber. The steam sterilization chamber may comprise an autoclave. The temperature within the steam sterilization chamber may typically be in the range from about 121°C to about 135°C. The biological indicator may be placed in the sterilization chamber in one or more locations where it is difficult for steam to reach to verify that the steam is penetrating these locations. Upon completion of the sterilization process, the biological indicator may be incubated in the presence of a growth media to determine whether the sterilization process is effective.

The biological indicator may comprise a carrier inoculated with an aqueous carbohydrate solution containing test organisms. The carrier may comprise a porous material or a non-porous material. The carrier may comprise a solid material. The carrier may comprise any material that does not dissolve or deteriorate during the sterilization or incubation processes. The carrier may comprise paper, metal, glass, ceramics, plastic, membranes, or a combination of two or more thereof. The metal may comprise aluminum or steel. The plastic may comprise a polyolefin, polystyrene, polycarbonate, polymethacrylate, polyacrylamide, polyimide, polyester, and the like. The carrier may comprise a film. The carrier may be in the form of a spun or unwoven felt. The carrier may comprise a mat of compressed fibers. The carrier may comprise a porous material made of sintered glass, glass fibers, ceramic, synthetic polymer, or a combination of two or more thereof. The carrier may comprise filter paper or absorbent paper. The carrier may comprise a cellulose pad.

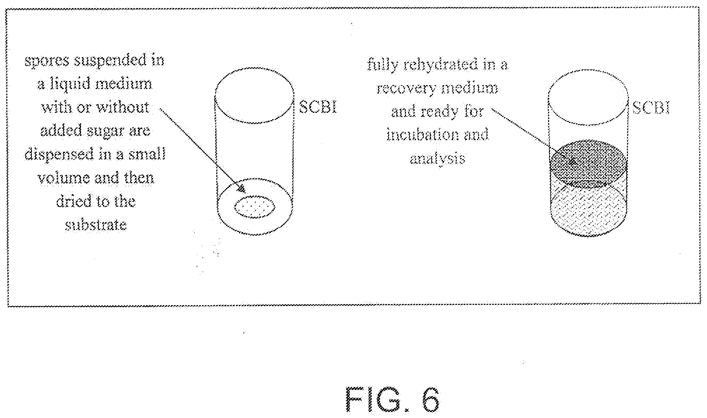

The carrier may be positioned in an SCBI. The carrier may comprise a surface in a compartment of an SCBI. The carrier or interior surface of the SCBI may be inoculated with an aqueous carbohydrate solution containing test organisms. A diagrammatic representation of this process is given in Fig. 6. The SCBI may comprise a capped container with two separate compartments. One of the compartments may contain the inoculated carrier or inoculated interior surface. The other compartment may contain a growth media. This compartment may comprise a frangible glass vial of growth media that can be broken just prior to incubation. In use, the SCBI and the articles to be sterilized are exposed to steam during a steam sterilization process. Then following sterilization, the SCBI may be activated to allow for the test organisms to come into contact with the growth media. The SCBI may then be incubated to determine whether the sterilization process is effective. The invention provides a self-contained biological indicator as defined in appended claims 1-6.

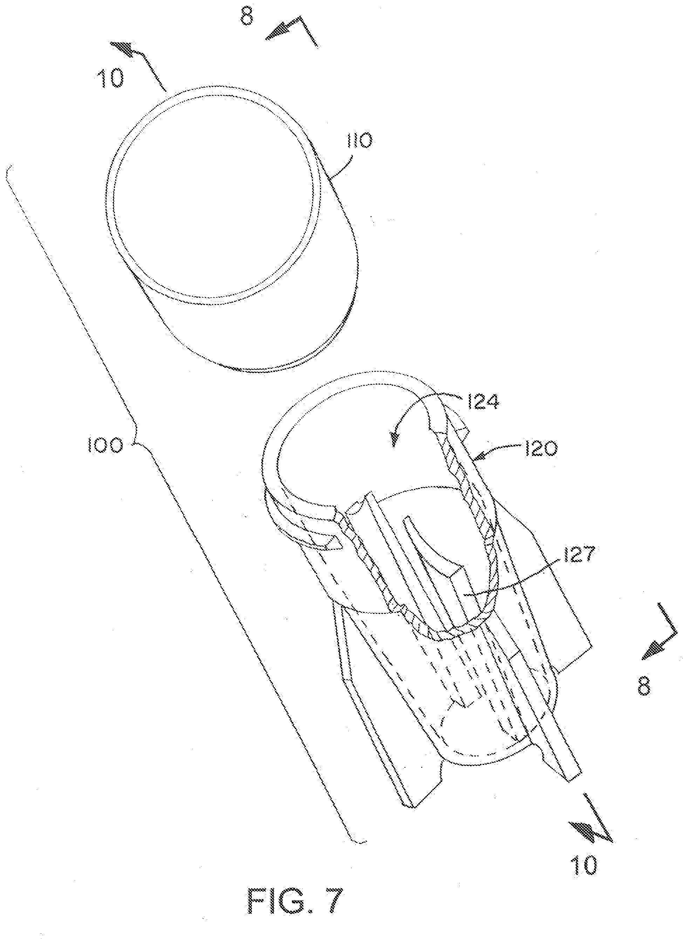

The SCBI may be in the form illustrated in Figs. 7-10. Referring to Figs. 7-10, SCBI 100 includes cap 110 which is configured for housing fluid 140. The fluid 140 contains a growth media. Cap 110, which is mounted on container 120, includes inner chamber 116. The inner chamber 116 has an opening 115 with a breakable barrier 130 overlying the opening 115. The fluid 140 is encapsulated within the inner chamber 116. The container 120 has an interior region 124 where carrier 190 is positioned. The carrier 190 is formed by inoculating it with a carbohydrate solution containing test organisms. The solution is dried to provide the inoculated carrier 190.

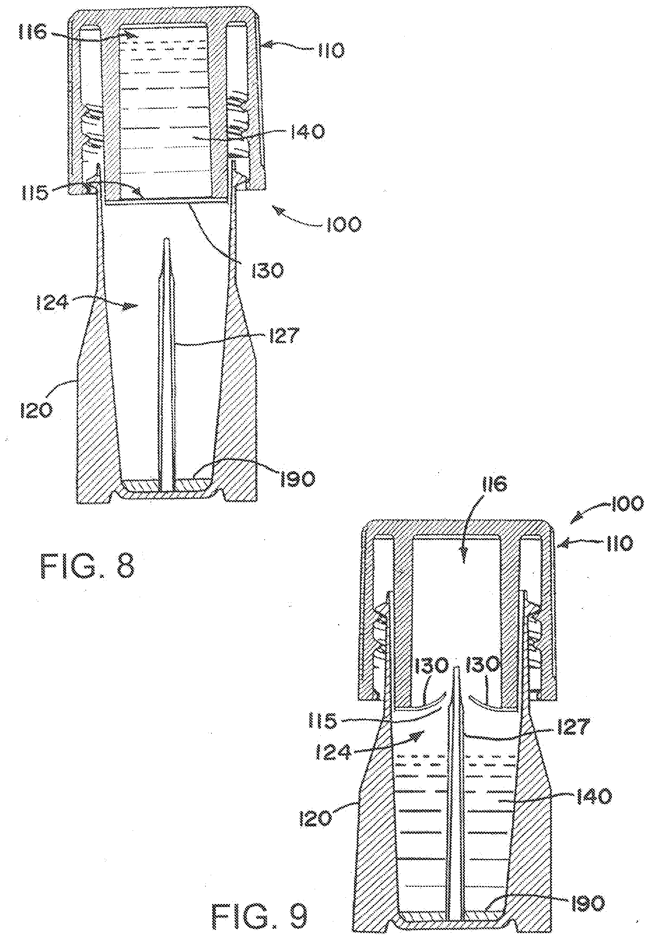

When used in a sterilization process, the cap 110 is held in an open position as illustrated in Fig. 8. The SCBI 100 and items to be sterilized are then subjected to the steam sterilization process. During the sterilization process, the steam passes through openings between the cap 110 and the container 120 and flows into the interior region 124 where it contacts and acts upon the test organisms deposited on the carrier 190.

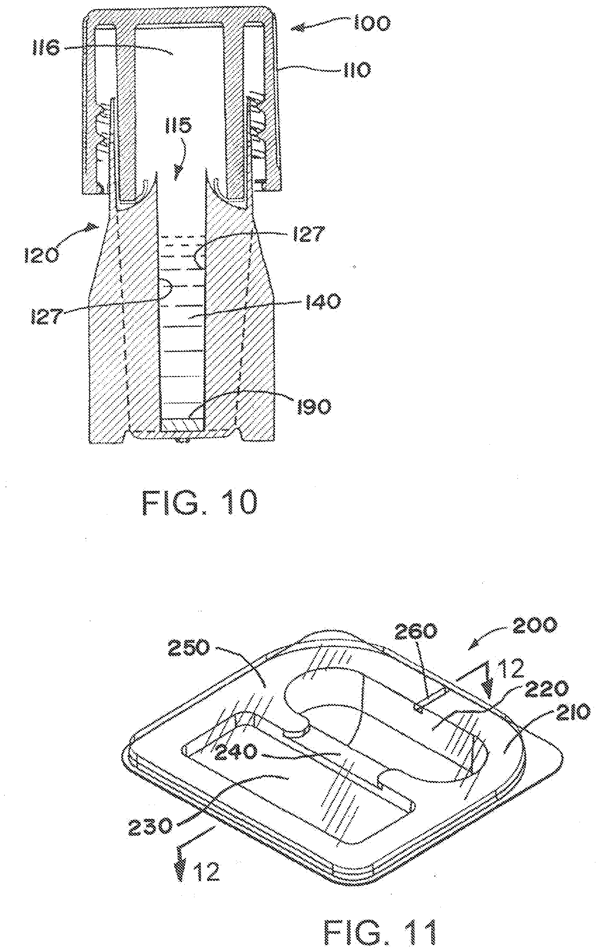

After the sterilization process is complete, the SCBI 100 is activated by screwing the cap 110 downward into a closed position as shown in Figs. 9 and 10. This results in the breakable barrier 115 being broken by puncture member 127 to form broken barrier 130. (Note that two puncture members may be used in the embodiment shown in Fig. 10). Fluid 140, which contains a growth media, then flows into the container 120 in contact with the test organisms deposited on the carrier 190.

While in container 120, the test organisms and growth media may be incubated for a sufficient period of time to determine the viability of the test organisms. At the end of the incubation period, the SCBI is evaluated to determine whether any test organisms survive the sterilization process. If the test organisms survive the sterilization process, the sterilization process is not considered to have been successful. On the other hand, if the test organisms are inactivated, then the sterilization process is considered to be successful.

A more detailed description of the SCBI 100 is disclosed in U.S. Patent 8,173,388. It should be noted that SCBI configurations other than those depicted in Figs. 7-10 may be used.

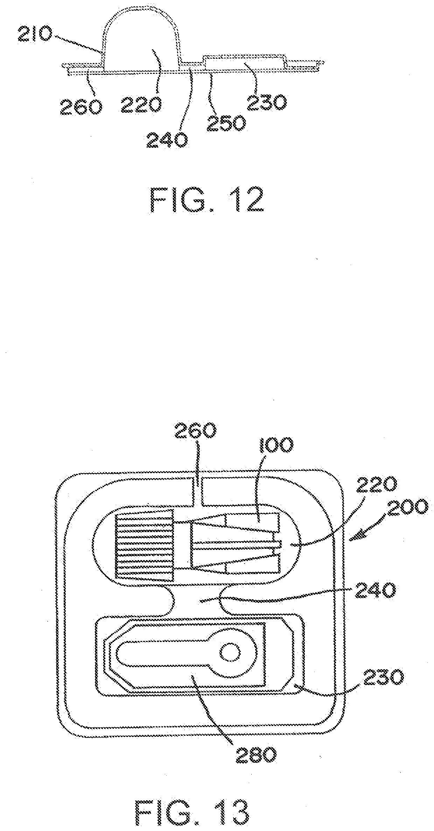

The SCBI 100 may be used with a test pack as depicted in Figs. 11-13. Referring to Figs. 11-13, test pack 200 includes base 210 containing recessed compartments 220 and 230. The recessed compartments 220 and 230 are in fluid communication with each other via internal channel 240. Cover 250 is attached to the base 210 and forms a sealed enclosure for the recessed compartments 220 and 230. External channel 260 provides a fluid communication between the sealed enclosure and an external environment. The SCBI 100 is positioned in recessed compartment 220. A chemical integrator and/or a chemical indicator 280 is positioned in recessed compartment 230. The external channel 260 is configured to allow a restricted flow of a steam sterilization medium into the recessed compartments 220 and 230. The base 210 and cover 250 are otherwise impenetrable by the steam sterilization medium. The chemical integrator and/or chemical indicator 280 undergoes changes in the position of a leading edge or in color after it has been exposed to a sufficient quantity of heat and steam in the sterilization medium for a sufficient period of time to indicate that the sterilization process has been completed. The steam sterilization medium also flows into the SCBI 100 in recessed compartment 220, and contacts the test organisms in the SCBI 100. A more detailed description of test pack 200 is disclosed in U.S. Patent 9,017,944 B2. It should be noted that test pack configurations other than those depicted in Figs. 11-13 may be used.

Another modification of the above forms of the biological indicator may be a rapid read or fast acting biological indicator. In this form the biological indicator may be mated to a dedicated instrument (reader) that detects early signals of test organism viability.

The carrier may be inoculated with an aqueous carbohydrate solution containing a suspension of the test organisms. The concentration of carbohydrate in the aqueous carbohydrate solution may range from about 1 to about 300 grams per liter(g/L), or from about 5 to about 150 g/L, or from about 10 to about 75 (g/L). The molar concentration for the carbohydrate in the aqueous carbohydrate solution may be in the range from about 0.001 M to about 10 M, or from about 0.01 M to about 1 M. The concentration of the test organism in the aqueous carbohydrate solution may range from about 10⁴ to about 10⁸ colony forming units (cfu) per milliliter (ml), or from about 10⁵ to about 10⁷ cfu/ml.

Referring to Fig. 6, a desired amount of spores in water may be spun down and resuspended in one of the carbohydrate solutions prior to being dispensed into vials and dried. For example, a suspension of *Geobacillus stearothermophilus* spores in a carbohydrate solution may be prepared to yield a desired number of spores per aliquot for inoculating the carrier. The test organisms may be dispensed and allowed to dry on the carrier. An air flow may be used to dry the test organisms on the carrier, such as, for example, by placing the carrier in a laminar flow-hood to hasten the drying process. The method of drying the test organisms on the carrier may include allowing the test organisms to air dry by leaving them stand under ambient conditions, placing the inoculated test organisms in a desiccator containing a desiccant such as calcium chloride, in a temperature and humidity controlled environmental chamber, or placing the inoculated BIs under a stream of dry air, nitrogen or other anhydrous gas. The number of colony forming units of the test organism supported by the carrier may be in the range from about 10⁴ to about 10⁷ cfu per square millimeter of support (cfu/mm²), or from about 10⁵ to about 10⁶ cfu/mm². When ready for use the SCBI may be placed in a sterilization chamber along with the materials to be sterilized and exposed to the sterilant. Once removed from the sterilization chamber the SCBI may be activated causing the release of the growth medium which rehydrates the spores. The SCBI may then be incubated for a prescribed period of time and monitored for survivors.

The biological indicator may be used to release loads or validate sterilization chamber functionality in healthcare settings. The biological indicator may also be used to determine if biological indicator waste has been properly decontaminated. In the scientific setting, the biological indicator may be used to validate the functionality of sterilization chambers, release loads of goods, or validate that a process meets required functionality. A valid biological indicator for a given process requires a specific resistance and therefore biological indicator manufacturers may strive to manufacture the biological indicator with targeted resistance characteristics.

To insure that the biological indicator lot is appropriate for its intended use, it may be characterized by comparison to a pre-determined resistance. For use in healthcare applications the pre-determined resistance may be established by the U.S. Environmental Protection Agency (EPA) or the U.S. Food and Drug Administration (FDA). For other non-regulated applications it may be established by customer preference. Even in the regulated applications it may be desirable to target a resistance at the low end of the mandated range.

The resistance for the biological indicator may be expressed as its D-value, which quantifies the time required to achieve inactivation of 90% of the population of test organisms. In the case of steam SCBIs the D-value may be measured in minutes. The D-value may be in the range from about 0.01 to about 5 minutes, or from about 0.1 to about 5 minutes.

Referring to Figs. 5A and 5B, the steam resistance of a biological indicator may be determined using BIER vessel 300. The BIER vessel can be used to quickly deliver a dose of steam to the load by controlling pressure, time, and temperature. The BIER vessel can also be used to quickly dissipate the dose. This allows for the resistance of a biological indicator to the selected steam sterilization process to be determined.

The resistance of a biological indicator may be targeted to meet customer and/or regulatory requirements. For example, a healthcare biological indicator may be required to meet a targeted D-value for a 121°C sterilization cycle of 1.5 - 3.0 minutes in a BIER. This resistance level may be required by regulatory guidance documents and expected by healthcare consumers. Other standard cycles may vary in the desired or regulated D-value range.

The production of biological indicators may be open to many variables. The specific strain of spore used, the process by which the selected test organism has been cultivated, the scale of cultivation performed, the materials of construction of the carrier and the constituents of the growth media or other ingredients used in the carrier are all components of the biological indicator that may impact resistance. Minor changes in these components may have significant effects upon the measured resistance characteristics of the biological indicator. This can lead to batches of biological indicators that do not meet the established resistance specifications.

In the prior art, the variability inherent to biological indicator fabrication has led to problems with the manufacturing process and an inability to deliver the desired final product to the customer. Variability in cultivation of the test organism, changes in suppliers of the materials of construction, or Iot-to-Iot variability in the components of the growth media are a few of the examples of very minor modifications that can lead to unwanted changes in resistance in a biological indicator line that is in production over a period of time.

Additionally, use of specific validated materials of construction in biological indicators allows for limited ability to alter the resistance of a given product to meet customer and regulatory requirements. For example, if a specific product has a D-value of 2.90 minutes in the required conformation, this product will be unlikely to meet the needs of a customer looking for a reduced resistance with a D-value of 1.90 minutes. Both these values may be within the mandated range. To prepare a product having a reduced resistance, the manufacturer may have to cultivate additional lots of test organisms in the hope that the inherent variability of the process would uncontrollably yield the desired lower resistance.

This invention relates to a biological indicator comprising a carrier which is inoculated with an aqueous carbohydrate solution containing a suspension of test microorganisms, as defined in claims 1-6. The introduction of the carbohydrate to the biological indicator is used to reduce the resistance of the biological indicator to steam sterilization. This allows for facilitated production of a biological indicator with a targeted reduced resistance to steam sterilization.

The biological indicator may be used by subjecting it to the same steam sterilization medium and treatment as the articles for which sterile conditions may be sought. Steam may pass into the area where the biological indicator is located thereby exposing the biological indicator to the same sterilization process as the articles being sterilized. Following sterilization, growth media may be brought into contact with the biological indicator. The growth media may be in the form of a liquid. The growth media may comprise a buffered aqueous solution. Any procedure whereby the biological indicator is brought into contact with the growth media under conditions which allow for growth of the test organisms, if it still exists, may be used. The growth media may be present in the sterilization chamber in powder or tablet form and, after sterilization, sterile water may be added such that the biological indicator comes into contact with the aqueous incubation medium.

The growth media may comprise one or more nutrient sources. The nutrient source may be used to provide energy for the growth of any of the test organisms that may survive the sterilization process. Examples of the nutrient sources may include pancreatic digest of casein, enzymatic digest of soybean meal, sucrose, dextrose, yeast extract, L-cystine, and mixtures of two or more thereof.

A microbial growth indicator, which changes color or native state, in the presence of viable test organisms may be used with the growth media. The growth indicator may be dispersed or solubilized in the growth media and impart an initial color to the growth media. The growth indicator may also impart a color change in the growth media upon test organism growth. Growth indicators which may be employed include pH-sensitive dye indicators (such as bromothymol blue, bromocresol purple, phenol red, etc. or combinations thereof), oxidation-reduction dye indicators (such as methylene blue, etc.). The use of these microbial growth indicators may result in a change in color in response to a phenomenon of microorganism growth, such as changes in pH, oxidation-reduction potentials, enzymatic activity, as well as other indications of growth.

The growth media may further comprise one or more pH buffers, one or more neutralizers, one or more agents for maintaining osmotic equilibrium, or a mixture of two or more thereof. The pH buffers may include K₂HPO₄, KH₂PO₄, (NH₄)₂HPO₄, 2,2-Bis(hydroxylmethyl)-2,2',2"-nitrilothiethanol (Bis Tris), 1, 3-Bis[tris(hydroxymethyl)methylamino] propane (Bis-Tris Propane), 4-(2-Hydroxyethyl)piperazine-ethanesulfonic acid (HEPES), 2-Amino-2-(hydroxymethyl)-1,3-propanediol (Trizma, Tris base), *N-*[Tris(hydroxymethyl)methyl]glycine (Tricine), Diglycine (Gly-Gly), *N,N*-Bis(2-hydroxyethyl)glycine (Bicine), *N*-(2-Acetamido)iminodiacetic acid (ADA), *N*-(2-Acetamido)-2-aminoethanesulfonic acid (aces), 1,4-Piperazinediethanesulfonic acid (PIPES), β-Hydroxy-4-morpholinepropanesulfonic acid (MOPSO), *N,N*-Bis(2-hydroxyethyl)-2-aminoethanesulfonic acid (BES), 3-(N-Morpholino)propanesulfonic acid (MOPS), 2-[(2-Hydroxy-1,1-bis(hydroxylmethyl)ethyl)amino]ethanesulfonic acid (TES), 3-(*N,N*-Bis[2-hydroxyethyl]amino)-2-hydroxypropanesulfonic acid (DIPSO), 4-(N-Morpholino)butanesulfonic acid (MOBS), 2-Hydroxy-3-[tris(hydroxymethyl)methylamino]-1-propanesulfonic acid (TAPSO), 4-(2-Hydroxyethyl)piperazine-1-(2-hydroxypropanesulfonic acid hydrate (HEPPSO), Piperazine-1,4-bis(2-hydroxypropanesulfonic acid) dihydrate (POPSO), 4-(2-Hydroxyethyl)-1-piperazine propanesulfonic acid (EPPS), *N*-(2-Hydroxyethyl)piperazine-N'-(4-butanesulfonic acid) (HEPBS), [(2-Hydroxy-1,1-bis(hydroxymethyl)ethyl)amino]-1-propanesulfonic acid (TAPS), 2-Amino-2-methyl-1,3-propanediol (AMPD), N-tris(Hydroxymethyl)methyl-4-aminobutanesulfonic acid (TABS), N-(1,1-Dimethyl-2-hydroxyethyl)-3-amino-2-hydroxypropanesulfonic acid (AMPSO), 2-(Cyclohexylamino)ethanesulfonic acid (CHES), 3-(Cyclohexylamino)-2-hydroxyl-1-propanesulfonic acid (CAPSO), 2-Amino-2-methyl-1-propanol (AMP), 3-(Cyclohexylamino)-1-propanesulfonic acid (CAPS), 4-(Cyclohexylamino)-1-butanesulfonic acid (CABS), 2-(*N*-Morpholino)ethanesulfonic acid hydrate (MES), *N*-(2-Acetamido)-2-aminoethanesulfonic acid (ACES), and mixtures of two or more thereof.

The neutralizers may include but are not limited to sodium thioglycollate, sodium thiosulfate, catalase, sodium bisulfate, sodium bisulfite lecithin, polysorbate 20, polysorbate 80, calcium bicarbonate, and mixtures of two or more thereof.

The agents for maintaining osmotic equilibrium may include sodium salt, potassium salts, magnesium salts, manganese salts, calcium salts, metallic salts, sodium chloride, potassium chloride, magnesium sulfate, iron chloride, and mixtures of two or more thereof.

The growth media may comprise an aqueous composition comprising: water; from about 0.01 to about 100 grams per liter (g/l), or from about 0.1 to about 50 g/l, of one or more nutrient sources; from about 1.0x10⁻⁵ to about 10 g/l, or from about 1.0x10⁻⁴ to about 1.0 g/l of one or more microbial growth indicators; up to about 5000 g/l, or from about 0.001 to about 5000 g/l, or from about 0.1 to about 1000 g/l, of one or more pH buffers; up to about 100 g/l, or from about 0.01 to about 100 g/l, or from about 0.1 to about 50 g/l, of one or more neutralizers; up to about 50 g/l, or from about 0.1 to about 50 g/l, or from about 0.1 to about 25 g/l, of one or more agents for maintaining osmotic equilibrium.

### Examples

In the following illustrative examples, the test organism is prepared using a standard lot of *Geobacillus stearothermophilus* spores suspended in an aqueous carbohydrate solution. In these examples, xylose and dextrose are used as the carbohydrates. Comparative examples are also provided wherein the carbohydrate is not used. In each case, the aqueous suspension is placed in a vial and allowed to dry. This is shown in Fig. 6. The resulting biological indicators are resistance tested. The tests show an unexpected decrease in resistance when the biological indicators are derived from a carbohydrate solution as compared to when no carbohydrate is used.

In these examples, the biological indicators are tested for resistance to steam sterilization using the biological indicator evaluation resistor (BIER) vessel depicted in Fig. 5A. Referring to Fig. 5A, BIER vessel 300 includes chamber 310, door 320, and interior shelf 330 for placement of the biological indicators. Steam, heat and pressure enters chamber 310 as indicated by arrow 340. Vent 350 is provided for the rapid release of steam, heat and pressure. Operation is controlled by a computerized processor, sensors, and valves designed to introduce rapid changes to the environment within the chamber 310. This results in the "square-wave" cycle characteristics depicted in Fig. 5B.

Referring to Fig. 5B, the process cycle used in the BIER vessel includes six phases, these phases being numbered as phases 1-6 in Fig. 5B. These phases can be described as follows:

| **Phase** | **Description** |
|---|---|
| Air removal (Phase 1) | Air is removed from the test chamber by evacuation to a selected vacuum level. Subatmospheric steam is injected into the test chamber so that the partial pressure remaining in the test chamber is steam. |
| Steam charge (Phase 2) | Steam is injected into the test chamber until the selected test temperature is attained. |
| Exposure time (Phase 3) | The test chamber is maintained at the selected test temperature for the selected exposure time. |
| Exhaust/ postvacuum (Phase 4) | The test chamber is evacuated below atmospheric pressure to a selected level. |
| Air vent (Phase 5) | The test chamber is vented to atmospheric pressure. |
| Cycle complete (Phase 6) | Test samples are manually removed from the chamber following completion of the cycle. |

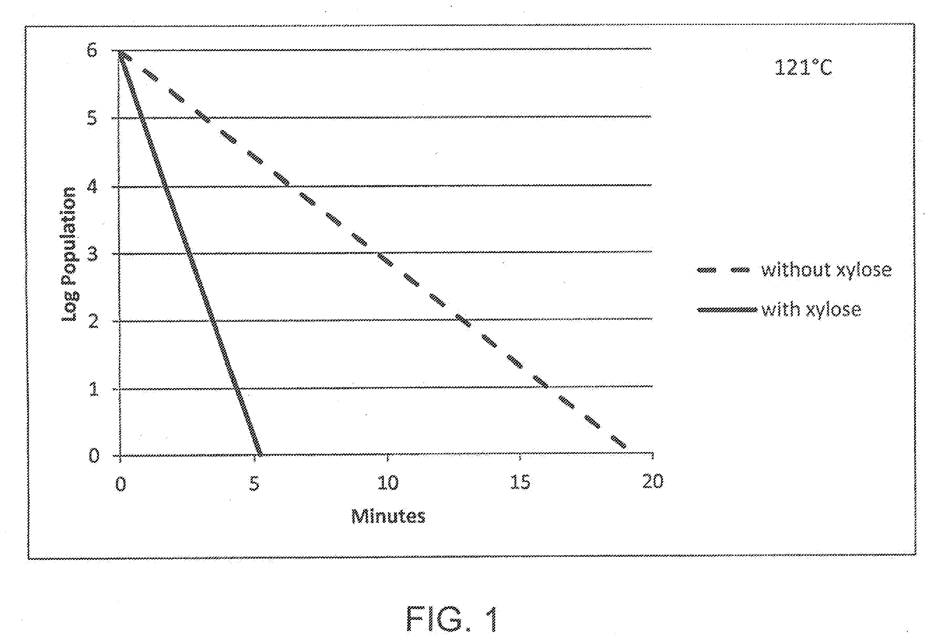

The results for the following Examples 1-4 are shown in Figs. 1-4. Each of Figs. 1-4 shows a plot of log population versus time in minutes. The term "log population" refers to the log of the number of colony-forming units (cfu) of viable spores in the test sample. For example, a log population of 6 refers to 10⁶ (or 1,000,000) viable colony-forming units of spores in the test sample.

### Example 1

The biological indicators are evaluated for resistance to steam at 121°C in the BIER vessel. A first set of biological indicators is derived from a suspension of *Geobacillus stearothermophilus* spores in water. A second set of biological indicators is derived from a suspension of the spores in a 1.67 M xylose solution. The resulting kill kinetics are significantly shorter when the second set of biological indicators derived from the xylose solution (a 6 log reduction in approximately 5 minutes) is used as compared to the first set of biological indicators where the xylose solution is not used (a 6 log reduction in approximately 20 minutes). This is shown in Fig. 1.

### Example 2

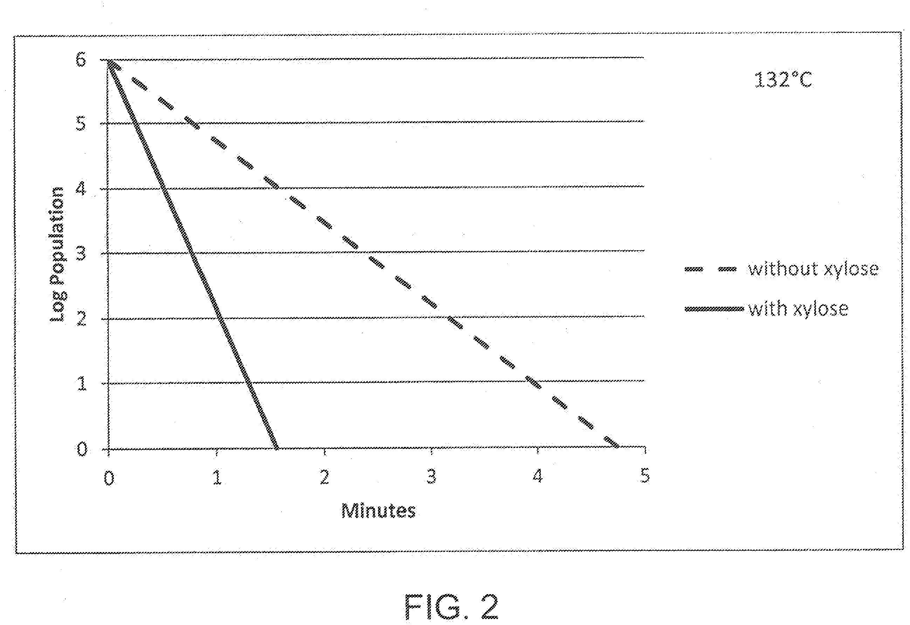

The tests conducted in Example 1 are repeated except that they are performed at 132°C in the BIER vessel. This testing shows a reduction in resistance from approximately 5 minutes (without xylose) to approximately 1.5 minutes (with xylose). The kill kinetics are faster at 132°C than at 121°C. The addition of xylose reduces resistance even further. This is shown in Fig. 2.

### Example 3

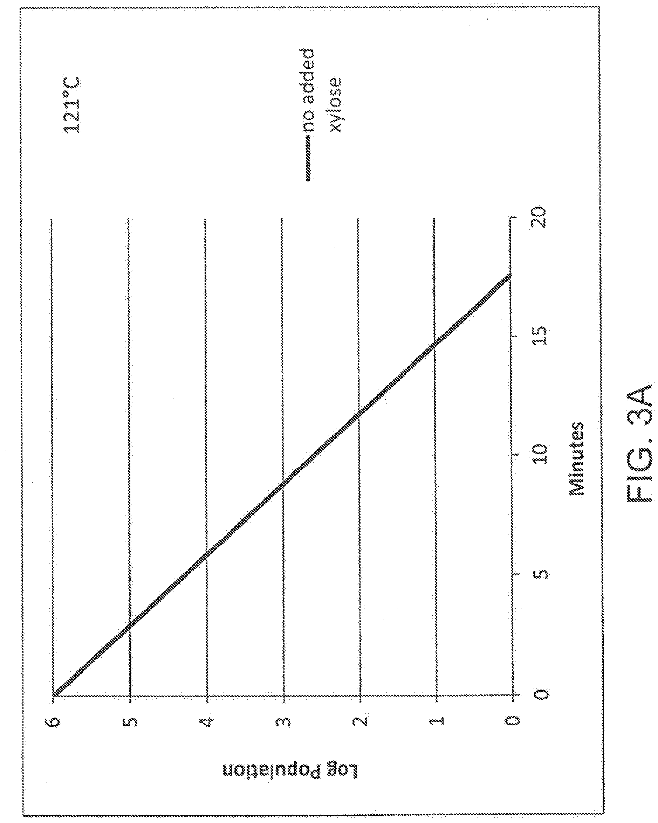

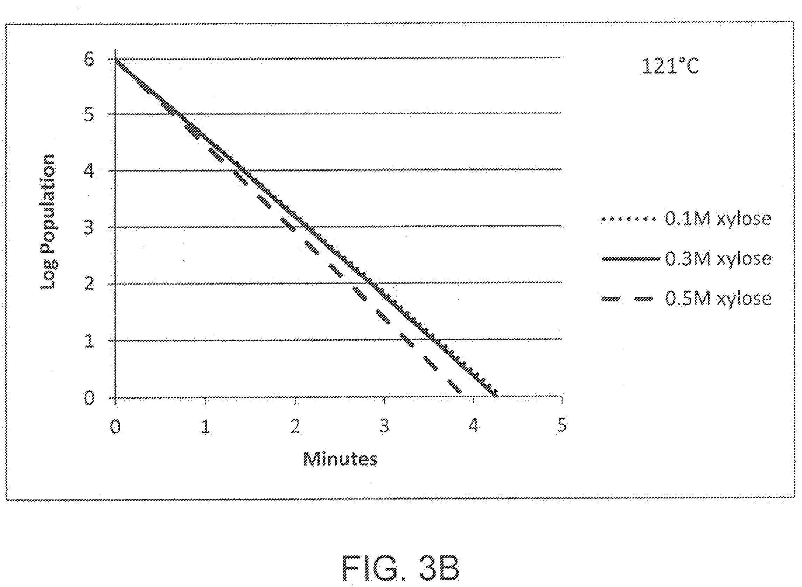

Additional testing is performed to evaluate the impact of the quantity of xylose that is used. The xylose concentration that is used ranges from no xylose being used (Fig. 3A) to a range of concentrations of xylose from 0.1M to 0.5M xylose solutions (Fig. 3B). The results shown a reduction from approximately 17.5 minutes (no xylose, Fig. 3A) to approximately 4 minutes (0.5M xylose, Fig. 3B).

### Example 4

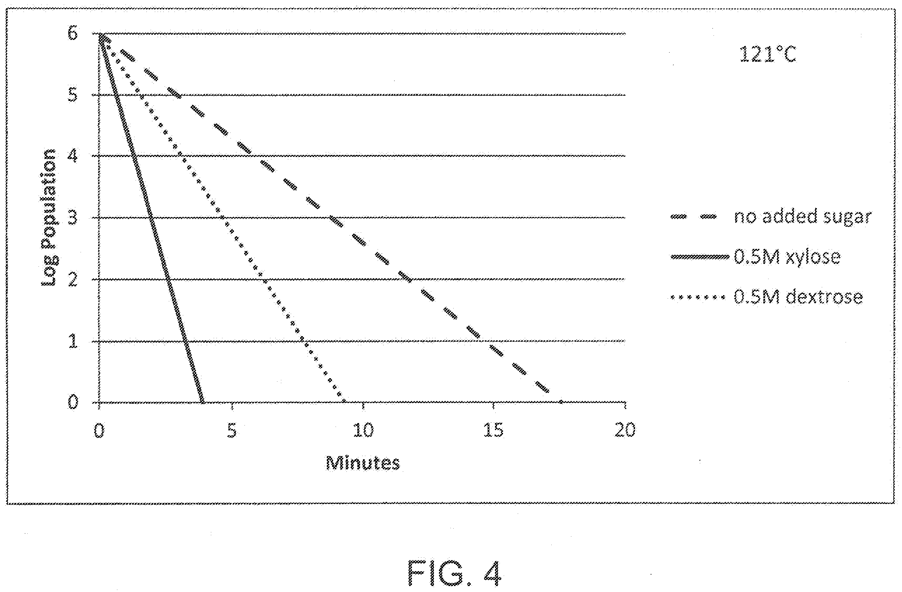

The tests conducted in Example 1 are repeated except that different carbohydrates are used. In these tests, biological indicators derived from suspensions of *Geobacillus stearothermophilus* spores in 0.5M solutions of xylose are compared to biological indicators derived from suspensions of the spores in 0.5M solutions of dextrose and biological indicators derived from suspensions of the spores in water without a carbohydrate being used. The results are shown in Fig. 4. These results show that the resistance for biological indicators derived from *Geobacillus stearothermophilus* spores suspended in water only is approximately 17.5 minutes, while the resistance of biological indicators derived from spores suspended in 0.5M solutions of xylose is approximately 4 minutes and the resistance for biological indicators derived from spores suspended in 0.5M solutuions of dextrose is approximately 9 minutes.

While the invention has been explained in relation to various embodiments, it is to be understood that various modifications thereof will become apparent to those skilled in the art upon reading the specification. Therefore, it is to be understood that the invention disclosed herein includes any such modifications that fall within the scope of the appended claims.

## Claims

1. A self-contained biological indicator, comprising: a carrier inoculated with test microorganisms and an effective amount of a carbohydrate to reduce the resistance of the biological indicator to steam sterilization as compared to when no carbohydrate is used, and wherein the carbohydrate comprises a monosaccharide selected from xylose or dextrose and the test microorganisms comprise *Geobacillus stearothermophilus* spores; and
wherein the carrier comprises an interior surface of a first compartment of the self-contained biological indicator, the carrier being inoculated by depositing an aqueous solution containing the carbohydrate and the test microorganisms on the carrier and drying the solution to form an inoculated carrier, the first compartment being adapted to permit the test microorganism to be brought into contact with steam during a steam sterilization process; and
the self-contained biological indicator further comprising a second compartment containing a growth media, the second compartment being adapted to maintain the growth media separate from the test microorganism during the steam sterilization process, and the second compartment being adapted to permit the growth media to contact the test microorganism after the steam sterilization process is completed.

2. The self-contained biological indicator of claim 1 wherein the self-contained biological indicator is positioned in a test pack.

3. The self-contained biological indicator of claim 1 or claim 2 wherein the carbohydrate comprises xylose.

4. The self-contained biological indicator of any of claims 1-3 wherein the molar concentration of the carbohydrate in the aqueous solution is in the range from 0.001 M to 10 M.

5. The self-contained biological indicator of claim 4 wherein the concentration of the test microorganisms in the aqueous solution is in the range from 10⁴ to 10⁷ cfu per milliliter.

6. The self-contained biological indicator of any of claims 1-5 wherein the number of test microorganisms on the carrier is in the range from 10⁴ to 10⁷ cfu/mm².

7. A steam sterilization process, comprising:
exposing an article to be sterilized and the self-contained biological indicator of any of claims 1-6 to steam.

8. A process for determining the effectiveness of a steam sterilization process, comprising:
exposing an article to be sterilized and the self-contained biological indicator of any of claims 1-6 to steam; and
incubating the test microorganisms in the presence of the growth media to determine whether the sterilization process is effective.

## Patentansprüche

1. In sich geschlossener biologischer Indikator, umfassend:
einen Träger, der mit Testmikroorganismen und einer wirksamen Menge eines Kohlenhydrats geimpft ist, um den Widerstand des biologischen Indikators gegenüber Dampfsterilisation im Vergleich dazu zu verringern, wenn kein Kohlenhydrat verwendet wird, und wobei das Kohlenhydrat ein Monosaccharid umfasst, ausgewählt aus Xylose oder Dextrose, und die Testmikroorganismen *Geobacillus stearothermophilus* Sporen umfassen; und
wobei der Träger eine Innenfläche einer ersten Kammer des in sich geschlossenen biologischen Indikators umfasst, wobei der Träger durch Abscheiden einer wässrigen Lösung, die das Kohlenhydrat und die Testmikroorganismen enthält, auf dem Träger, und Trocknen der Lösung geimpft wird, um einen geimpften Träger zu bilden, wobei die erste Kammer angepasst ist, um zuzulassen, dass der Testmikroorganismus während eines Dampfsterilisationsvorgangs in Kontakt mit Dampf gebracht wird; und
wobei der in sich geschlossene biologische Indikator ferner eine zweite Kammer umfasst, die ein Wachstumsmedium enthält, wobei die zweite Kammer angepasst ist, um das Wachstumsmedium getrennt von dem Testmikroorganismus während des Dampfsterilisationsvorgangs zu halten, und wobei die zweite Kammer angepasst ist, um zuzulassen, dass das Wachstumsmedium den Testmikroorganismus kontaktiert, nachdem der Dampfsterilisationsvorgang beendet ist.

2. In sich geschlossener biologischer Indikator nach Anspruch 1, wobei der in sich geschlossene biologische Indikator in einer Testpackung positioniert ist.

3. In sich geschlossener biologischer Indikator nach Anspruch 1 oder Anspruch 2, wobei das Kohlenhydrat Xylose umfasst.

4. In sich geschlossener biologischer Indikator nach einem der Ansprüche 1-3, wobei die Molkonzentration des Kohlenhydrats in der wässrigen Lösung im Bereich von 0,001 M bis 10 M liegt.

5. In sich geschlossener biologischer Indikator nach Anspruch 4, wobei die Konzentration der Testmikroorganismen in der wässrigen Lösung im Bereich von 10⁴ bis 10⁷ KBE pro Milliliter liegt.

6. In sich geschlossener biologischer Indikator nach einem der Ansprüche 1-5, wobei die Anzahl von Testmikroorganismen auf dem Träger im Bereich von 10⁴ bis 10⁷ KBE/mm² liegt.

7. Dampfsterilisationsvorgang, umfassend:
Aussetzen eines Artikels, der sterilisiert werden soll, und des in sich geschlossenen biologischen Indikators nach einem der Ansprüche 1-6, unter Dampf.

8. Verfahren zum Bestimmen der Wirksamkeit eines Dampfsterilisationsvorgangs, umfassend:
Aussetzen eines Artikels, der sterilisiert werden soll, und des in sich geschlossenen biologischen Indikators nach einem der Ansprüche 1-6, unter Dampf; und
Inkubieren der Testmikroorganismen in Anwesenheit des Wachstumsmediums, um zu bestimmen, ob der Sterilisationsvorgang wirksam ist.

## Revendications

1. Indicateur biologique autonome, comprenant : un support inoculé avec des microorganismes de test et une quantité efficace d'un glucide pour réduire la résistance de l'indicateur biologique à la stérilisation à la vapeur par rapport au cas où aucun glucide n'est utilisé, et dans lequel le glucide comprend un monosaccharide choisi parmi le xylose ou le dextrose et les microorganismes de test comprennent des spores de *Geobacillus stearothermophilus* ; et
dans lequel le support comprend une surface intérieure d'un premier compartiment de l'indicateur biologique autonome, le support étant inoculé en déposant une solution aqueuse contenant le glucide et les microorganismes de test sur le support et en séchant la solution pour former un support inoculé, le premier compartiment étant adapté pour permettre au microorganisme de test d'être mis en contact avec de la vapeur pendant un processus de stérilisation à la vapeur ; et l'indicateur biologique autonome comprenant en outre un second compartiment contenant un milieu de croissance, le second compartiment étant adapté pour maintenir le milieu de croissance séparé du microorganisme de test pendant le processus de stérilisation à la vapeur, et le second compartiment étant adapté pour permettre au milieu de croissance de contacter le microorganisme de test une fois le processus de stérilisation à la vapeur terminé.

2. Indicateur biologique autonome selon la revendication 1, dans lequel l'indicateur biologique autonome est positionné dans un module de test.

3. Indicateur biologique autonome selon la revendication 1 ou la revendication 2, dans lequel le glucide comprend du xylose.

4. Indicateur biologique autonome selon l'une quelconque des revendications 1 à 3, dans lequel la concentration molaire du glucide dans la solution aqueuse est dans la plage de 0,001 M à 10 M.

5. Indicateur biologique autonome selon la revendication 4, dans lequel la concentration des microorganismes de test dans la solution aqueuse est dans la plage de 10⁴ à 10⁷ cfu par millilitre.

6. Indicateur biologique autonome selon l'une quelconque des revendications 1 à 5, dans lequel le nombre de microorganismes de test sur le support est dans la plage de 10⁴ à 10⁷ cfu/mm².

7. Procédé de stérilisation à la vapeur, comprenant :
l'exposition d'un article à stériliser et de l'indicateur biologique autonome selon l'une quelconque des revendications 1 à 6 à la vapeur.

8. Procédé pour déterminer l'efficacité d'un procédé de stérilisation à la vapeur, comprenant :
l'exposition à la vapeur d'un article à stériliser et de l'indicateur biologique autonome selon l'une quelconque des revendications 1 à 6 ; et
l'incubation des microorganismes de test en présence du milieu de croissance pour déterminer si le processus de stérilisation est efficace.
